# EUROPEAN PATENT SPECIFICATION

(11) **EP 4 522 990 B1**
(45) Date of publication and mention of the grant of the patent: **25.03.2026**
(21) Application number: 23728612.5
(22) Date of filing: 10.05.2023
(51) Int. Cl.: G01N 33/574, G01N 33/68

(54) **USE OF BMMF1 REP PROTEIN AS DIAGNOSTIC MARKER FOR PANCREATIC CANCER AND DIABETES MELLITUS TYPE 2**
VERWENDUNG DES BMMF1 REP-PROTEINS ALS DIAGNOSTISCHER MARKER FÜR BAUCHSPEICHELDRÜSENKREBS UND DIABETES MELLITUS TYP 2
UTILISATION DE PROTÉINE REP BMMF1 EN TANT QUE MARQUEUR DE DIAGNOSTIC DE CANCER DU PANCRÉAS ET DU DIABÈTE SUCRÉ DE TYPE 2

(30) Priority: 11.05.2022 EP 22172801
(43) Date of publication of application: 19.03.2025
(73) Proprietor: Deutsches Krebsforschungszentrum Stiftung des öffentlichen Rechts, 69120 Heidelberg (DE)
(72) Inventor: BUND, Timo, 69120 Heidelberg (DE); ZUR HAUSEN, Harald, 69120 Heidelberg (DE); DE VILLIERS-ZUR HAUSEN, Ethel-Michele, 69120 Heidelberg (DE); ALIKHANYAN, Kristina, 69120 Heidelberg (DE); ERNST, Claudia, 69120 Heidelberg (DE); TESSMER, Claudia, 69120 Heidelberg (DE); GIESE, Nathalia, 69198 Schriesheim (DE)
(74) Representative: Schüssler, Andrea
(86) International application number: PCT/EP2023/062464
(87) International publication number: WO 2023/217886

(56) References cited:
- EP-A1- 2 910 645
- EP-A1- 3 208 614
- EP-A1- 3 517 960
- EP-A1- 3 699 594
- EP-A1- 3 699 595
- WO-A1-2011/064179
- WO-A1-2015/162532
- WO-A2-2004/031105
- WO-A2-2016/005054
- BUND TIMO ET AL: "Analysis of chronic inflammatory lesions of the colon for BMMF Rep antigen expression and CD68 macrophage interactions", PROCEEDINGS OF THE NATIONAL ACADEMY OF SCIENCES, vol. 118, no. 12, 15 March 2021 (2021-03-15), XP055973710, ISSN: 0027-8424, DOI: 10.1073/pnas.2025830118
- HARALD ZUR HAUSEN ET AL: "Dairy cattle serum and milk factors contributing to the risk of colon and breast cancers", INTERNATIONAL JOURNAL OF CANCER, JOHN WILEY & SONS, INC, US, vol. 137, no. 4, 20 February 2015 (2015-02-20), pages 959 - 967, XP071289276, ISSN: 0020-7136, DOI: 10.1002/IJC.29466
- ZUR HAUSEN HARALD ET AL: "Infectious Agents in Bovine Red Meat and Milk and Their Potential Role in Cancer and Other Chronic Diseases", CURRENT TOPICS IN MICROBIOLOGY AND IMMUNOLOGY, SPRINGER BERLIN HEIDELBERG, BERLIN, HEIDELBERG, vol. 407, 1 January 2017 (2017-01-01), pages 83 - 116, XP009514829, ISSN: 0070-217X, ISBN: 978-3-540-92165-3, DOI: 10.1007/82_2017_3
- HARALD ZUR HAUSEN ET AL: "Specific nutritional infections early in life as risk factors for human colon and breast cancers several decades later", INTERNATIONAL JOURNAL OF CANCER, JOHN WILEY & SONS, INC, US, vol. 144, no. 7, 6 December 2018 (2018-12-06), pages 1574 - 1583, XP071290651, ISSN: 0020-7136, DOI: 10.1002/IJC.31882
- DE VILLIERSZUR HAUSEN: "Bovine Meat and Milk Factors (BMMFs): Their proposed role in common human cancers and type 2 diabetes mellitus", CANCERS (BASEL, vol. 13, no. 21, 2021, pages 5407, XP002807831
- ETHEL-MICHELE DE VILLIERS ET AL: "A specific class of infectious agents isolated from bovine serum and dairy products and peritumoral colon cancer tissue", EMERGING MICROBES & INFECTIONS, vol. 8, no. 1, 1 January 2019 (2019-01-01), pages 1205 - 1218, XP055627144, DOI: 10.1080/22221751.2019.1651620

## Description

The invention relates to the use of a DNA-replication-associated (Rep) protein as a diagnostic marker for pancreatic cancer and diabetes mellitus type 2.

### Background of the Invention

The pancreas serves as an exocrine gland that secretes pancreatic juice which is transported to the digestive tract through the pancreatic duct, while also functioning as an endocrine gland that secretes hormones such as insulin and glucagon into blood. Thus, diseases of the pancreas are often a cause of or are closely connected with diabetes.

Since the pancreas is surrounded by many organs such as the stomach, the duodenum, the small intestine, the liver, and the gallbladder, pancreatic cancer is not only difficult to detect early but has properties such as a lack of subjective symptoms, very rapid progression, and metastasis to other organs and thus has very poor prognosis as compared with other cancers. Despite several new cancer treatment options, e.g. immunotherapeutic approaches with checkpoint inhibitors or bispecific antibodies, the 5-year relative survival rates by cancer types remain lowest with between 6% and 10% in pancreatic cancer

The basic therapy of pancreatic cancer is practiced by surgery, systemic chemotherapy, radiotherapy, or a combination thereof depending on a stage of progression. Although 15 to 20% pancreatic cancer patients undergo surgery for potential complete cure, the great majority of patients will not benefit from surgery due to local progression or metastasis.

The UICC (Unio Internationalis Contra Cancrum) stages of progression of pancreatic cancer are classified into stages 0, IA, IB, IIA, IIB, III, IVa, and IVb. Stages I to III occupy half or more of the number of 5-year survivals, and stages IVa and IVb occupy 70% or more of the stages of progression at the time of diagnosis. Therefore, early detection and treatment of pancreatic cancer are necessary.

Abdominal ultrasonography is a very useful, convenient and minimally invasive examination method in check-ups for patient care or medical examination. However, it is often difficult to visualize pancreatic cancer having a small tumor size or a lesion on the pancreatic tail side. In medical check-ups, the abnormality detection rate with pancreatic images by abdominal ultrasonography is approximately 1%, and the detection rate of pancreatic cancer is approximately 0.05% or lower.

Meanwhile, cystic diseases that occur in the pancreas are known to progress to invasive cancers through malignant transformation and can be regarded as pancreatic cancer precursor lesions. The malignancy of the cystic diseases is evaluated on the basis of cyst diameters, wall thickening, diameters of the main pancreatic duct, mural nodules, stenosis of the main pancreatic duct, enlarged lymph nodes, and cystic lesions, etc. Patients with mucinous cystic tumors (also called mucinous cystadenom), one type of benign cystic disease, have a high probability for developing malignant cancer and are therefore recommended to receive regular follow-ups or tumor resection, even if there is no malignancy of the neoplasms when they are detected.

To enhance earlier detection, there is a need for biomarkers that will facilitate early detection and further insights into the pathogenesis of pancreatic cancer.

EP 3 208 614 A1 relates to a composition and method for diagnosing pancreatic cancer, wherein e.g. CA19-9, LRG1, TTR, C1R or KLKB1 have been used as markers.

Bund et al., 2021 discloses the analysis of chronic inflammatory lesions of the colon for BMMF Rep antigen expression and CD68 macrophage interactions.

Zur Hausen and de Villiers et al., 2015 analyse epidemiological data as regards the question whether diary cattle serum and milk factors may contribute to the risk of colon and breast cancers.

EP 2 910 645 A1 refers to the assessment of cancer patients for their benefit from treatment with antibodies against tumor cell antigens by determining macrophage lineage cell levels in tumor samples.

WO 2011/064179 A1 provides OLFM4 as a marker protein for the early detection of type II diabetes, antibodies directed to the marker protein and their use in a diagnostic method for type II diabetes and in drug development.

### Description of the Present Invention

The inventors found that the uptake of BMMF (Bovine Meat and Milk Factor) agents within the first months of life either by substitution of breast-feeding during weaning by cow milk products or by the uptake of dairy or beef products, in general, leads to the early infection of newborns with BMMF antigens. The early exposure is thereby hypothesized to induce an immunological state in which the individuals will not clear or neutralize BMMF which is taken up via the nutrition in the following part of life as efficiently as individuals which profited from a longer breastfeeding period. Within the next years or decades - depending on the immune system of the host additional BMMF antigens accumulate within the stroma of pancreatic tissue. This accumulation may be triggered also by the uptake of specific molecules that represent receptors for BMMFs. These molecules are also taken up by consumption of cow products and are incorporated into lectin receptors on the surface of the host cells. When a certain level of antigen is reached by continuous uptake of BMMFs in combination with focal spreading of infection, the host immune response induces a state of chronic and local inflammation producing a stable increase of diffusing, reactive oxygen species (ROS) and cyclooxygenase-2 (Cox-2) which dramatically increases the probability of deregulated cell proliferation with concomitant fixation of random mutations in surrounding cells induced by ROS (de Villiers and zur Hausen, 2021). Especially, cells with intrinsically high replicative activity might represent targets enriching random DNA mutations enabling stochastic manifestation of mutations as a basic requirement for tumorigenesis and development of pancreatic cancer. Thus, BMMFs represent a specific and local trigger for induction of chronic inflammation within the tissue stroma leading to an increase of ROS which induces proliferation and mutation in surrounding replicative cells eventually leading to the formation of hyperplasia as precursors for cancer.

The invention is defined by the appended claims.

In detail, structural properties of pleomorphic vesicles, regularly identified by staining tissues of prostate cancer patients for expression of Rep protein of a subgroup of BMMF1, were observed. The latter represent proteins involved in replication of small single-stranded circular plasmids of BMMF, but most likely also contributing to pleomorphic vesicular structures found in the periphery of pancreatic cancers. Gold-labelled, structurally dense regions are demonstrated in preselected areas of pancreatic cancer, after staining with gold-labelled monoclonal antibodies against BMMF1 Rep. Similar structures were observed in human embryonic cells (HEK293TT) overexpressing Rep. These data suggest that Rep or Rep isoforms contribute to the structural formation of vesicles. Additionally, a co-localization of the anti-Rep-stained signals with CD68-positive macrophages was observed. The regions with highest Rep-specific antibody detection correlate with regions with highest detection levels for CD68 positive cells pointing towards a localization of the Rep-specific antigens in inflammatory tissue areas, i.e. regions with especially high levels of inflammatory monocytes, circulating macrophages, or resident tissue macrophages.

Bovine sera were initially subjected to density gradient centrifugation. Single fractions of these gradients were analysed for structural particles by electron microscopy, as well as DNA extraction, amplification and sequencing. Comparison to all databanks revealed a DNA sequence homology to the transmissible spongiform encephalopathy (TSE)-associated isolates Sphinx 1.76 (1,758 bp; accession no. HQ444404) and Sphinx 2.36 (2,360 bp; acc no. HQ444405) (Manuelidis L. 2011). Full-length plasmid-like, episomal DNA genomes of Bovine Meat and Milk Factors (BMMF) were obtained using abutting primers and PCR amplification on the partial DNA fragments. The main open reading frame of all isolates was identified *in silico* as a putative replication gene (Rep). and revealed a remarkable degree of similarity to specific plasmids of *Acinetobacter baumannii* and Psychrobacter species. More than 130 BMMF DNA genomes were subsequently isolated from milk and other dairy products, as well as 3 isolates from a brain biopsy and serum from multiple sclerosis patients. These isolates were grouped as BMMF1, BMMF2 and BMMF4 based on DNA sequence homology (Funk et al. 2014, Gunst et al. 2014, Whitley et al. 2014, Falida et al., 2017, de Villiers et al., 2019). Detailed analyses of their genomes revealed characteristics of both viruses and bacterial plasmids needed for replication, transcription and translation, frequently all present in a single genome, thereby constituting a novel class of pathogens (de Villiers et al. 2019).

Two BMMF1 isolates from a brain biopsy of a MS patient were MSBI1.176 (MSBI, multiple sclerosis brain isolate) (1,766 bp) and MSBI2.176 (1,766 bp) which are designated as "MSBI1 genome" and "MSBI2 genome", respectively. MSBI1.176 shares 98% nucleotide similarity to the sequence of Sphinx 1.76. The sequences of the isolates MSBI1.176 and MSBI2.176 have been deposited in the EMBL Databank under accession numbers LK931491 (MSBI1.176) and LK931492 (MSBI2.176) (Whitley C. et al. 2014) and have been aligned and described in WO 2016/005054 A2.

Further isolates were obtained from cow milk, dairy products and bovine sera. These Cow milk isolates (CMI) were CMI1.252, CMI2.214, CMI3.168, CMI4.158, HCBI3.108 ,HCBI4.296, HCBI6.252, CMI.M3.1 and CMI.9M.1 which are designated as "CMI1 genome", "CMI2 genome", "CMI3 genome", "CMI4 genome", "HCBI3 genome", "HCBI4 genome", "HCBI6 genome", "C1M1.3M.1 genome", "C1MI.9M.1 genome", respectively. The sequences of the isolates have been deposited in the EMBL Databank under accession numbers LK931487 (CMI1.252), LK931488 (CMI2.214), LK931489 (CMI3.168), LK931490 (CMI4.158), LK931495 (HCBI3.108), LK931496 (HCBI4.296), LK931493 (HCBI6.252), LR215499 (C1MI.3M.1), LR215496 (C1MI.9M.1) and have been aligned and described in part in WO 2016/005054 A2. The CMI5 genomes are described in Falida et al., 2017.

The present inventors have found that both CMI genomes and MSBI genomes replicate and show a significant production of transcribed RNA in vitro. Antibodies against the MSBI1.176 Rep show expression in the peripheral tissue around the pancreatic cancer tissue. The present inventors have found that the encoded Rep proteins of a subgroup of BMMF1 infectious agents (MSBI1 Rep, MSBI2 Rep, CMI1 Rep, CMI2 Rep, CMI3 Rep, CMI4 Rep, HCBI3 Rep, HCBI4 Rep, HCBI6 Rep, C1MI.3M.1 Rep, C1MI.9M.1 Rep) represent a diagnostic marker for pancreatic cancer.

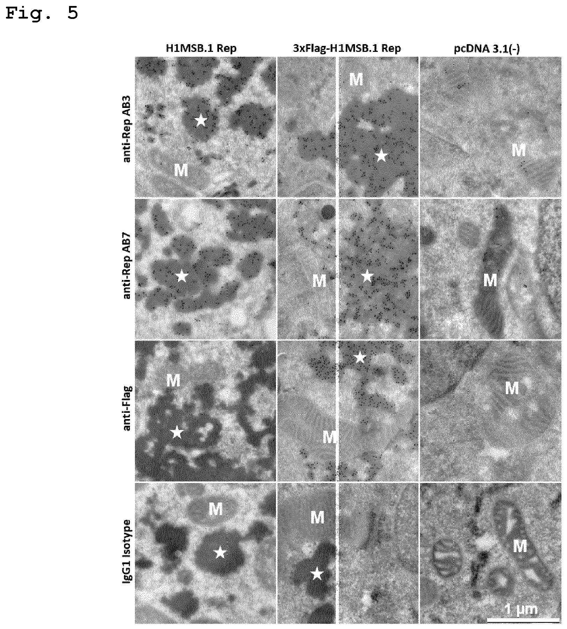

The inventors have raised monoclonal antibodies against Rep protein using full-length MSBI1.176 Rep or consensus peptides derived from two conserved regions of the MSBI1.176 Rep sequence. In particular embodiments, the anti-Rep antibodies bind to epitopes of Rep protein that are exemplified in Fig. 5. Particularly preferred antibodies bind to epitopes within an amino acid sequence selected from the group consisting of amino acids from 1 to 136, from 137 to 229 and from 230 to 324 of SEQ ID NO:1. For example, the antibody binds to an epitope comprised by SEQ ID NO:2 or SEQ ID NO:3.

### Brief description of the Figures:

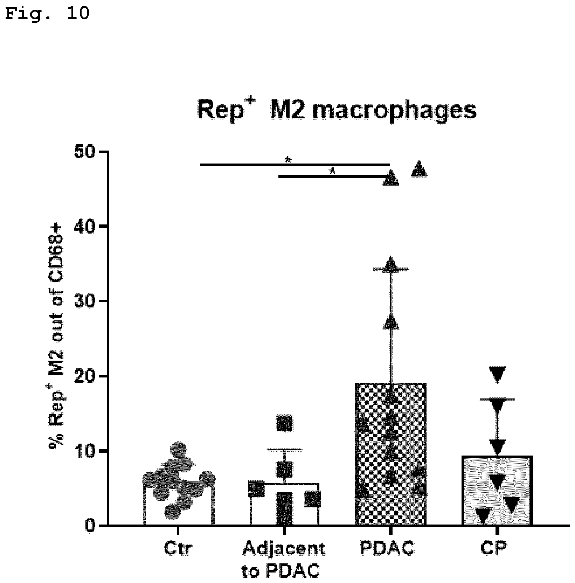

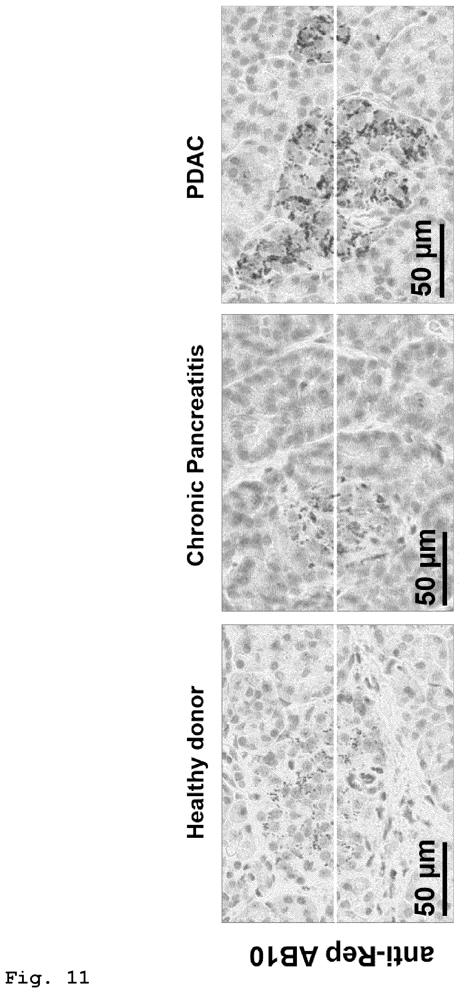

- Figure 1: **IHC detection of BMMF1 Rep on PDAC cancer patient tissue in consecutive tissue sections in CD68-enriched tissue regions (A) and in insulin-positive beta cells of Langerhans islands (B). Ctrl** = **Control**
- Figure 2: **Ultrastructural representation of BMMF in tissues of a pancreatic cancer patient.**
**(A)** IHC DAB staining of tissue peritumor pancreatic cancer tissue stained with anti-Rep AB3. Both endocrine cells (I, beta cells in Langerhans islets) and cells in the exocrine tissue region (II) are stained. **(B)** Pre-IEM of a pancreatic cancer FFPE biopsy of the same donor: Exocrine (upper row) and endocrine (low row) regions of peritumor pancreatic cancer tissue stained with AB3 showing gold-decorated pleomorphic structures.

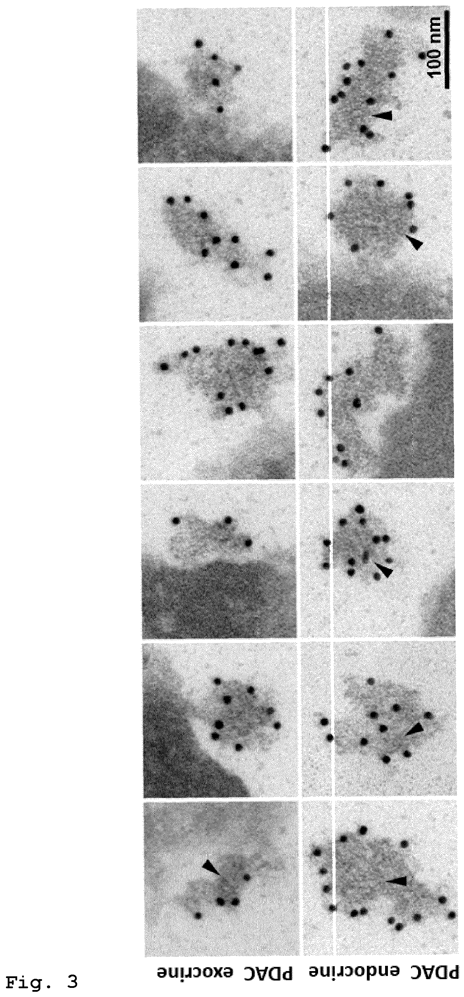

- Figure 3: **Ultrastructural representation of BMMF in tissues a pancreatic cancer patient stained with gold-labelled anti-Rep AB3 after pre-embedding immuno-electron microscopy (pre IEM)**
BMMF pleomorphic vesicles in human FFPE biopsies of exocrine and endocrine peritumor pancreatic cancer tissue regions. The tissues exhibit small, approximately 100 nm-sized pleomorphic bodies of a structurally-dense material that heavily stains with gold-labelled BMMF antibody. Even though no structural motif was identified, filamentous arrangements at sites indicate higher-order organization (arrowheads). Size of gold grains: 10 nm.

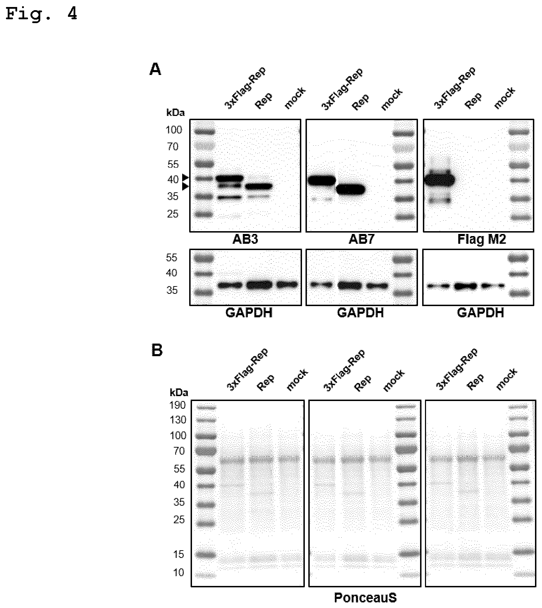

- Figure 4: **MSBI1.176 Rep overexpression in HEK293TT analyzed by western blotting.**
3xFlag- H1MSB.1 Rep (expected molecular size of 40 kDa, arrowhead) or a non-tagged Rep (37 kDa, arrowhead) were overexpressed in HEK293TT for 48 h and analyzed by SDS-PAGE and western blotting with anti-Rep (AB3 and AB7) and anti-Flag antibodies. Mock control transfection was performed with pcDNA3.1 (-) vector without Rep gene insert and GAPDH served as loading control. **(B)** PonceauS protein staining of the respective membranes.
- Figure 5: **Ultrastructural demonstration by cryo-IEM of BMMF expression in HEK293TT.**
Cells were transfected with either a plasmid for expression of H1MSB.1 Rep, 3xFlag-H1MSB.1 Rep, or the empty vector for control. Rep overexpression leads to formation of large segregation structures (asterisks) which stain intense with anti-Rep AB3 and AB7 and, in case of overexpressed 3xFlag-Rep, also with anti-Flag. These structures did not stain with the isotype control antibody. No such structures formed in cells transfected with the empty vector. Size of gold grains: 10 nm; M: mitochondrion.

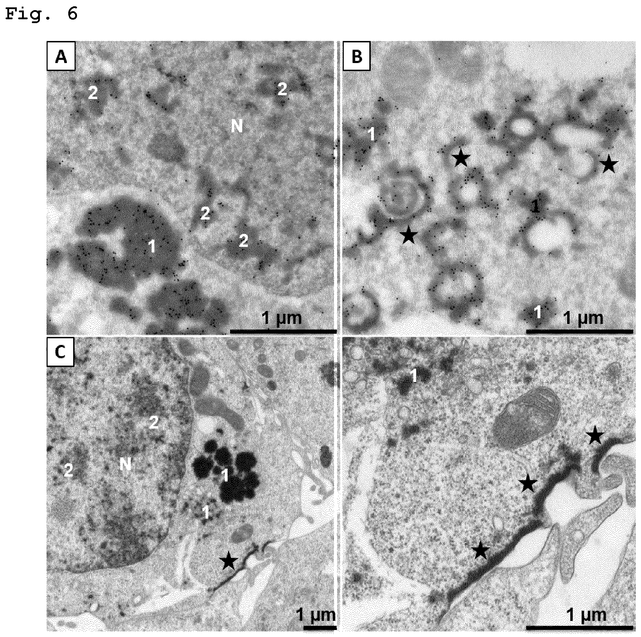

- Figure 6: **Membrane-associated BMMF in HEK293TT cells after overexpression of MSBI1.176 Rep.**
HEK293TT cells transiently transfected with a MSBI1.176 Rep expression plasmid (untagged, codon optimized) express partitioned pleiomorphic segregation structures which stain densely with AB7 (cryo IEM, **A** and **B),** as well as **(C)** with uranyl and lead contrasting agents for conventional resin TEM. Segregation structures typically occupy the cytoplasm of cells **(1)** and occasionally also the nucleoplasm **(2).** In addition, similar material accumulates along cellular membranes **(C** enlarged views to the right, asterisks). Size of gold grains: 10 nm; N: nucleus, M: mitochondrion.

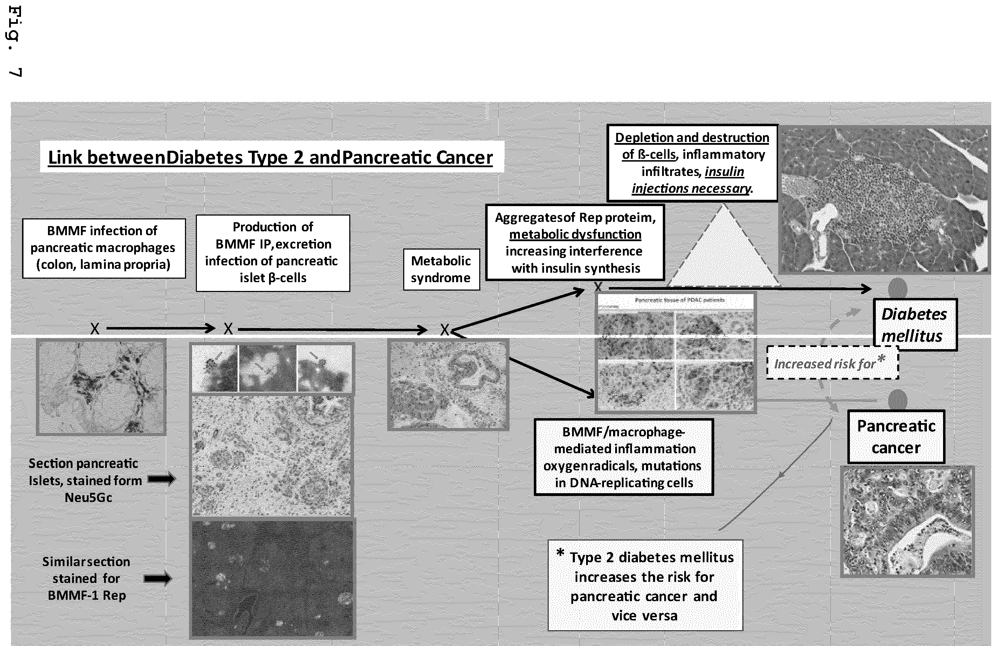

- Figure 7: **Modell conception of the etiology of pancreatic cancer and diabetes mellitus type 2 and their mutual relationship to each other**

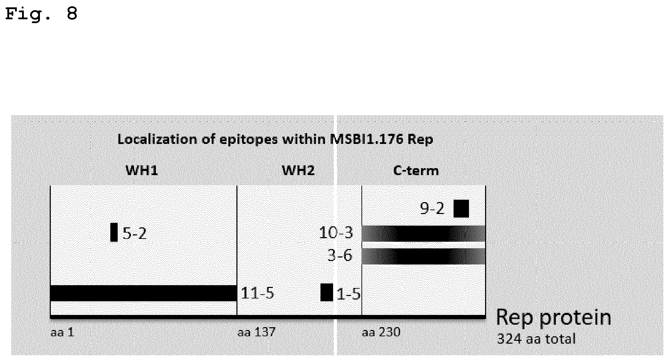

- Figure 8: **Localisation of the AB epitopes within MSBI1.176 Rep**

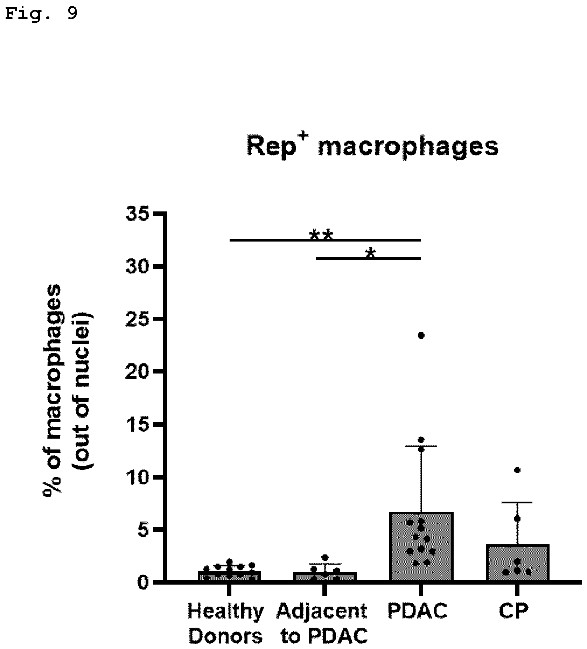

- Figure 9: **Quantification of Rep, CD68, and CD163 immunofluorescence microscopic staining**
Semi-automated, cell-based quantification of Rep, CD68, and CD163 immunofluorescence microscopic staining of individual FFPE tissues from tumour (PDAC, n=20) and pathologists-validated tumour-adjacent tissues of pancreatic ductal adenocarcinoma patients (n=6) and from non-cancer individuals (healthy donors, n=17) as well as tissues from chronic pancreatitis patients (CP, n=6). Mean representation of Rep+CD68+ cells in the respective tissues. Test with one-way ANOVA, Tukey's multiple comparisons. Significance: *P < .05, **P < .01, ***P < .001, ****P < .0001
- Figure 10: **Quantification of Rep+CD68+CD163+ cells of individual FFPE tissues**
Semi-automated, cell-based quantification of Rep+CD68+CD163+ cells of individual FFPE tissues from tumour (PDAC, n=20) and pathologists-validated tumour-adjacent tissues of pancreatic ductal adenocarcinoma patients (adjacent to PDAC, n=6) and from non-cancer individuals (Ctr, n=17) as well as tissues from chronic pancreatitis patients (CP, n=6). Mean representation of Rep+CD68+CD163+ cells within all macrophage cells in the respective tissues. Test with one-way ANOVA, Tukey's multiple comparisons. Significance: *P < .05, **P < .01, ***P < .001,****P < .0001
- Figure 11: **Immunohistochemical staining**
Immunohistochemical staining of representative FFPE tissue of PDAC, chronic pancreatitis and healthy donors based on anti-Rep AB10-3 in (staining with diaminobenzidine (DAB), scale bar: 50 µm).

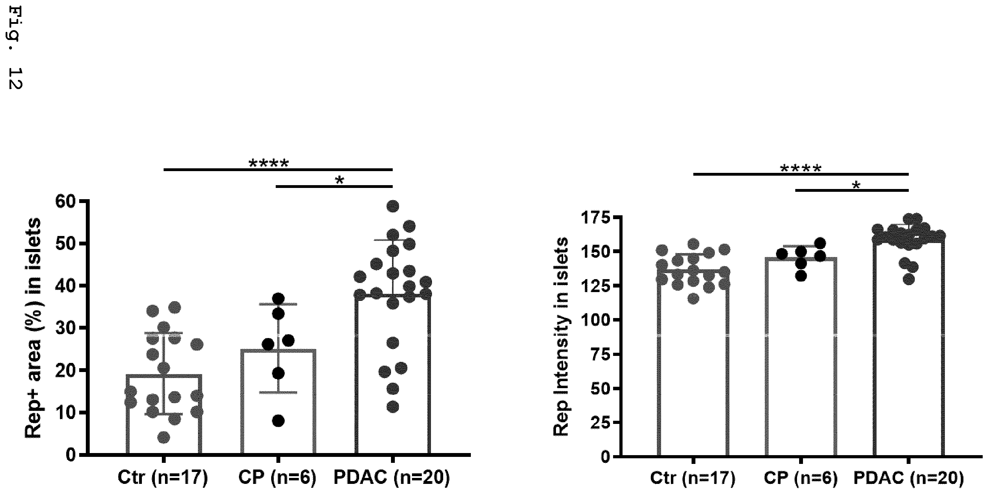

- Figure 12: **Semi-automated quantification of Rep-positive area and Rep staining intensity**
Semi-automated quantification of Rep-positive area (%) and Rep staining intensity (a.u.) in Langerhans islets of healthy donors (Ctr, n=17), chronic pancreatitis patients (CP, n=6) and pancreatic ductal adenocarcinoma patients (PDAC, n=20) (representation of mean). Tested by on-way ANOVA, Tukey's multiple comparisons. Significance: *P < .05, ****P < .0001

The invention provides the teaching that Rep proteins may represent diagnostic markers for an enhanced risk to develop pancreatic cancer or to diagnose pancreatic cancer or to confirm a pancreatic cancer diagnosis.

The terms "pancreatic cancer" means a cancer that evolved as a consequence of uncontrolled cell growth in the pancreatic gland. These malignancies may develop as a consequence of pre-existing benign cysts and hyperplasia where genetic alterations promote the transition from normal to cancerous growth. The term "pancreatic cancer" means pre-stages, early stages or late stages of the disease and metastases derived therefrom. "Pancreatic cancer" embraces all malignant neoplasms of the pancreas head, pancreas body, pancreas tail or of the Ductus pancreaticus. It may concern the exocrine or endocrine cells and embraces all types of exocrine or endocrine neoplasms. The most common exocrine tumors are pancreatic ductal adenocarcinoma (PDAC), tumors of the Papilla Vateri, intraductal papillary-mucinous tumor (IPMT) or acinus cell carcinoma. The most common endocrine tumors are insulinoma, gastinoma, somatostatinoma or glucagenoma.

In an alternative embodiment the present invention may also encompass the systematic testing of healthy pancreatic tissue (tissue from individuals without cancer diagnosis or a specific hint for the disease) to assess the disease risk in the future. This means that the present invention is also suitable to determine the predisposition for developing pancreatic cancer by use of the BMMF Rep as an early diagnostic marker.

"Predisposition" means, according to the accepted meaning in medicine, the tendency to a medicinal condition. It is the medicinal susceptibility to a specific disease or a condition of special susceptibility to a disease, usually based on the combined effects of genetic and environmental factors.

"Rep protein" as used herein refers to a DNA-replication-associated protein. The Rep protein comprises DNA binding activity and could be essential for initiation of replication of episomal/viral DNA molecules. In general Rep protein refers to a Rep protein from the group of BMMF1 proteins (de Villiers et al., 2019). In particular, the Rep protein is a MSBI1 genome-encoded Rep protein (MSBI1 Rep), a MSBI2 genome-encoded Rep protein (MSBI2 Rep), a CMI1 genome-encoded Rep protein (CMI1 Rep), a CMI2 genome-encoded Rep protein (CMI2 Rep), CMI3 genome-encoded Rep protein (CMI3 Rep), CMI4 genome-encoded Rep protein (CMI4 Rep), HCBI4 genome-encoded Rep protein (HCBI4 Rep), HCBI6-encoded Rep protein (HCBI6 Rep), C1MI.3M.1 genome-encoded Rep protein (C1MI.3M.1 Rep), C1MI.9M.1 genome-encoded protein (C1MI.9M.1 Rep). Preferably, the MSBI1 Rep protein is encoded by MSBI1.176 deposited in the EMBL databank under the acc. no. LK931491 and has the amino acid sequence as depicted in SEQ ID NO:1 or the Rep protein is MSBI2 encoded by MSBI2.176 deposited in the EMBL databank under the acc. no. LK931492 and has the amino acid sequence as depicted in SEQ ID NO:8 (Whitley, Gunst et al. 2014). In another preferred embodiment the CMI1 Rep protein is encoded by CMI1.252 deposited in the EMBL databank under the acc. no. LK931487 and has the amino acid sequence as depicted in SEQ ID NO:10. In another preferred embodiment the CMI2 Rep protein is encoded by CMI2.214 deposited in the EMBL databank under the acc. no. LK931488 and has the amino acid sequence as depicted in SEQ ID NO:11. In another preferred embodiment the CMI3 Rep protein is encoded by CMI3.168 deposited in the EMBL databank under the acc. no. LK931489 and has the amino acid sequence as depicted in SEQ ID NO:12. In another preferred embodiment the CMI4 Rep protein is encoded by CMI4.158 deposited in the EMBL databank under the acc. no. LK931490 and has the amino acid sequence as depicted in SEQ ID NO:16. In another preferred embodiment the HCBI3 is encoded by HCBI3.108 deposited in the EMBL databank under the acc. no. LK931495 and has the amino acid sequence as depicted in SEQ ID NO:17. In another preferred embodiment the HCBI4 is encoded by HCBI4.296 deposited in the EMBL databank under the acc. no. LK931496 and has the amino acid sequence as depicted in SEQ ID NO:18. In another preferred embodiment the C1MI.3M.1 is deposited in the EMBL databank under the acc. no. LR215499 and has the amino acid sequence as depicted in SEQ ID NO:19. In another preferred embodiment the C1MI.9M.1 is deposited in the EMBL databank under the acc. no. LR215496 and has the amino acid sequence as depicted in SEQ ID NO:20.

In a particular preferred embodiment the Rep protein comprises a N-terminal region conserved among BMMF1 genomes consisting essentially of amino acids from 1 to 229 of SEQ ID NO:1 and a C-terminal variable region specific for MSBI1.176 consisting essentially from amino acids 230 to 324 of SEQ ID NO:1. The N-terminal conserved region comprises a putative, first DNA binding domain consisting essentially of amino acids from 1 to 136 of SEQ ID NO: 1 and a second putative DNA binding domain consisting essentially of amino acids from 137 to 229 of SEQ ID NO:1. The C-terminal domain shows little sequence homology with any known protein and consists of amino acids 230 to 324.

"Rep protein" also encompasses fragments and variants of the protein with SEQ ID NO:1, SEQ ID NO:8, SEQ ID.NO:10, SEQ ID. NO: 11, SEQ ID. NO:12, SEQ ID.NO: 16, SEQ ID.NO:17, SEQ ID NO:18, SEQ ID NO:19 and/or SEQ ID.NO:20 which are capable of binding an anti-Rep antibody specific for Rep protein having the amino acid sequence of SEQ ID NO:1.. Preferably, such a fragment is an immunogenic fragment of the protein having the amino acid sequence of SEQ ID NO:1, SEQ ID NO:8, SEQ ID.NO:10, SEQ ID. NO: 11, SEQ ID. NO:12, SEQ ID.NO: 16, SEQ ID.NO:17, SEQ ID NO:18, SEQ ID NO:19 and/or SEQ ID.NO:20 which encompasses at least one epitope for an anti-Rep protein antibody against the Rep protein of SEQ ID NO:1. and, preferably, comprises at least 7, 8, 9, 10, 15, 20, 25 or 50 contiguous amino acids. In particular embodiments, the fragment comprises or consists essentially of a domain of the Rep protein, for example, the N-terminal conserved region, the C-terminal variable region, the first or second DNA binding domain. A variant of the protein with SEQ ID NO:1, SEQ ID NO:8, SEQ ID.NO:10, SEQ ID. NO: 11, SEQ ID. NO:12, SEQ ID.NO: 16, SEQ ID.NO:17, SEQ ID NO:18, SEQ ID NO:19 and/or SEQ ID.NO:20 comprises one or more amino acid deletions, substitutions or additions compared to respective sequence and has a homology of at least 90%, 91%, 92%, 93%, 94%, 95%, 96%, 97%, 98% or 99% to the amino acid sequence of SEQ ID NO:1, SEQ ID NO:8, SEQ ID.NO:10, SEQ ID. NO: 11, SEQ ID. NO:12, SEQ ID.NO: 16, SEQ ID.NO:17, SEQ ID NO:18, SEQ ID NO:19 and/or SEQ ID.NO:20, wherein the variant is capable of binding an anti-Rep antibody specific for a Rep protein having the amino acid sequence of SEQ ID NO:1. Included within the definition of variant are, for example, polypeptides containing one or more analogues of an amino acid (including, for example, unnatural amino acids, peptide nucleic acid (PNA), etc.), polypeptides with substituted linkages, as well as other modifications known in the art, both naturally occurring and non-naturally occurring. The term Rep protein includes fusion proteins with a heterologous amino acid sequence, with a leader sequence or with a Tag-sequence and the like. In certain embodiments of the invention protein tags are genetically grafted onto the Rep protein described above, for example the Rep protein selected from the group consisting of MSBI1, MSBI2, CMI1, CMI2, CMI3, CMI4, HCBI4, HCBI6, C1MI.3M.1 or C1MI.9M.1. In particular at least one protein tag is attached to a polypeptide consisting of an amino acid sequence as depicted in any one of SEQ ID NOs:1-3, 8-12, 14 and 16-20. Such protein tags may be removable by chemical agents or by enzymatic means. Examples of protein tags are affinity or chromatography tags for purification. For example, the Rep protein may be fused to a Tag-sequence, for example, selected from the group consisting of His₆-Tag (SEQ ID NO:4), T7-Tag (SEQ ID NO:5), FLAG-Tag (SEQ ID NO:6) and Strep-II-Tag (SEQ ID NO:7). a His-Tag (SEQ ID No:4), a T7-Tag (SEQ ID NO:5), FLAG-Tag (SEQ ID NO:6) or StrepII-Tag (SEQ ID NO:7). Further, fluorescence tags such as green fluorescence protein (GFP) or its variants may be attached to a Rep-protein according to the invention.

In a particular preferred embodiment, the MSBI1 genome-encoded Rep protein (MSBI1 Rep) is codon-optimized for the production in human cell lines (e.g. HEK-293, HEK293TT, HEK293T, HEK293FT, HaCaT, HeLa, SiHa, CaSki, HDMEC, L1236, L428, BJAB, MCF7, Colo678) as well as bovine (e.g. MAC-T) or murine cell lines (e.g. GT1-7) or any other cell line. This is described in detail in PCT/EP2017/075774.

The Rep protein of the invention, including the Rep fragments and Rep variants as defined above, can be prepared by classical chemical synthesis. The synthesis can be carried out in homogeneous solution or in solid phase. The polypeptides according to this invention can also be prepared by means of recombinant DNA techniques. "Subject" as used herein refers to a mammalian individual or patient, including murine, cattle, for example different bovine species, simians and humans. Preferably, the subject is a human patient.

"Anti-Rep antibody" as used herein refers to an antibody binding at a detectable level to Rep protein which affinity is more strongly to the Rep protein of the invention than to a non-Rep protein. Preferably, the antigen affinity for Rep protein is at least 2-fold, more preferably 5-fold or 10-fold, larger than background binding. In particular, the anti-Rep antibody detects the MSBI1 Rep having the amino acid sequence of SEQ ID NO:1. In particular embodiments, the antibody detects MSBI1 Rep, MSBI2 Rep, CMI1 Rep, CMI2 Rep, CMI3 Rep, CMI4 Rep, HCBI4 Rep, HCBI6 Rep, C1MI.3M.1 Rep, and/or C1MI.9M.1 Rep. In certain embodiments the anti-Rep antibody detects at least two, preferably all, of MSBI1 Rep, MSBI2 Rep, CMI1 Rep, CMI2 Rep, CMI3 Rep, CMI4 Rep HCBI4 Rep, HCBI6 Rep, C1MI.3M.1 Rep, and/or C1MI.9M.1 Rep.

The inventors also tested the antibody level of pancreatic cancer patients by contacting the Rep protein with a specimen suspected of containing anti-Rep protein antibodies under conditions that permit the Rep protein to bind to any such antibody present in the specimen. Such conditions will typically be physiologic temperature, pH and ionic strength using an excess of Rep protein. The incubation of the Rep protein with the specimen is followed by detection of immune complexes comprised of the antigen. In certain embodiments either the Rep protein is coupled to a signal generating compound, e.g. detectable label, or an additional binding agent, e.g. secondary antihuman antibody, coupled to a signal generating compound is used for detecting the immune complex.

Anti-Rep antibodies can be detected and quantified in assays based on Rep protein as protein antigen, which serves as target for the mammalian, e.g. human, antibodies suspected in the specimen. Preferably, the Rep protein is purified, and the specimen can be, for example, serum or plasma. The methods include immobilization of Rep protein on a matrix followed by incubation of the immobilized Rep protein with the specimen. Finally, the Rep-bound antibodies of the formed immunological complex between Rep protein and antibodies of the specimen are quantified by a detection binding agent coupled to a signal generating compound, e.g. secondary HRP-(horseradish-peroxidase)-coupled detection antibody allowing for HRP-substrate based quantification. This signal generating compound or label is in itself detectable or may be reacted with an additional compound to generate a detectable product.

Design of the immunoassay is subject to a great deal of variation, and many formats are known in the art. Protocols may, for example, use solid supports, or immunoprecipitation. Most assays involve the use of binding agents coupled to signal generating compounds, for example labelled antibody or labelled Rep protein; the labels may be, for example, enzymatic, fluorescent, chemiluminescent, radioactive, or dye molecules. Assays which amplify the signals from the immune complex are also known; examples of which are assays which utilize biotin and avidin or streptavidin, and enzyme-labelled and mediated immunoassays, such as ELISA assays.

The immunoassay may be in a heterogeneous or in a homogeneous format, and of a standard or competitive type. Both standard and competitive formats are known in the art.

In an immunoprecipitation or agglutination assay format the reaction between the Rep protein and the anti-Rep antibody forms a network that precipitates from the solution or suspension and forms a visible layer or film of precipitate. If no anti-Rep antibody is present in the specimen, no visible precipitate is formed.

In further embodiments the inventors used methods wherein an increased amount of Rep protein in a sample correlates with a diagnosis or predisposition of pancreatic cancer. In such embodiments the Rep protein in the sample is detected by anti-Rep antibodies.

"Sample" as used herein refers to a biological sample encompassing cancerous pancreatic tissue, peripheral tissue surrounding the cancerous tissue and (benign) hyperplasia. The samples encompass tissue samples such as tissue cultures or biopsy specimen.

Such methods are ex-vivo or in-vitro and comprise the steps of detecting Rep protein in an isolated sample from a subject by anti-Rep antibodies. In such methods Rep protein is detected in tissue samples by immunohistochemical methods or immunofluorescence microscopy.

In certain embodiments anti-Rep antibodies are used for the detection or capturing of the Rep protein in the sample.

The term "antibody", preferably, relates to antibodies which consist essentially of pooled polyclonal antibodies with different epitope specificities, as well as distinct monoclonal antibody preparations. As used herein, the term "antibody"(Ab) or "monoclonal antibody" (Mab) is meant to include intact immunoglobulin molecules as well as antibody fragments (such as, for example, Fab and F(ab')2 fragments) which are capable of specifically binding to Rep protein. Fab and F(ab')2 fragments lack the Fc fragment of intact antibody, clear more rapidly from the circulation, and may have less non-specific tissue binding than an intact antibody. Thus, these fragments are preferred, as well as the products of a FAB or other immunoglobulin expression library. Moreover, antibodies useful for the purposes of the present invention include chimeric, single chain, multifunctional (e.g. bispecific) and humanized antibodies or human antibodies.

In certain embodiments the antibody or antigen binding fragment thereof is coupled to a signal generating compound, e.g., carries a detectable label. The antibody or antigen binding fragment thereof can be directly or indirectly detectably labelled, for example, with a radioisotope, a fluorescent compound, a bioluminescent compound, a chemiluminescent compound, a metal chelator or an enzyme. Those of ordinary skill in the art will know of other suitable labels for binding to the antibody, or will be able to ascertain such, using routine experimentation.

Anti-Rep antibodies are, preferably, raised (generated) against a Rep protein having the amino acid sequence of SEQ ID NO: 1 or a fragment thereof by methods well known to those skilled in the art.

In certain embodiments anti-Rep antibodies are used in the methods of the invention which are capable of binding to several or all kinds of Rep proteins from the group of the BMMF1 proteins. Such antibody binds to an epitope within the conserved N-terminal region of the Rep protein from amino acids 1 to 229 of SEQ ID NO:1. In particular embodiments anti-Rep antibodies of the anti-BMMF1 Rep type are used which bind to an epitope within SEQ ID NO:2 (amino acids 32-49 of SEQ ID NO:1) or SEQ ID NO:3 (amino acids 197-216 of SEQ ID NO:1). The peptide fragments of SEQ ID NO:2 and SEQ ID NO:3 are highly conserved among the Rep proteins from BMMF1 and appear to be exposed due to their hydrophilic character. Anti-Rep antibodies of this anti-BMMF1 type may be produced by immunization, for example of mice or guinea pig, by peptides consisting essentially of the amino acid sequences as depicted in SEQ ID NOs:2 or 3; or by other immunogenic fragments, preferably comprising at least 8-15 amino acids, derived from the conserved N-terminal Rep protein region from amino acids 1 to 229 of SEQ ID NO:1.

In further embodiments anti-Rep antibodies specific for MSBI1 Rep protein are used. Such antibodies may be produced, for example, by immunization of a mammal such as mice or guinea pig with a full-length Rep protein having the amino acid sequence of SEQ ID NO:1.

Preferably, the methods of the invention use anti-Rep antibodies which are capable of detecting Rep protein up to ranges from pictogram to femtogram.

Examples of such groups of anti-Rep antibodies are shown in Table 1:

| **Antibody Group** | **Rep-Protein Localization** | **Detection** | **Antibody** | **DSMZ deposit** |
|---|---|---|---|---|
| **Group A** | cytoplasm + nuclear membrane (+nucleus) | BMMF1 Reps | AB01 523-1-1 (Ab 1-5) | DSM ACC3327 |
| **Group B** | speckles in cytoplasm | BMMF1 Reps | AB02 304-4-1 (Ab 5-2) | DSM ACC3328 |
| **Group C** | cytoplasm + nuclear membrane (+ nucleus) | MSBI1 Rep | MSBI1 381-6-2 (Ab 3-6) | DSM ACC3329 |
| | | | MSBI1 572-13-19 (Ab 10-3; AB10) | |
| | | | MSBI1 617-1-3 (Ab 11-5) | |
| **Group D** | speckles in cytoplasm | MSBI1 Rep | D1: MSBI1 961-2-2 (Ab 9-2) | DSM ACC3331 |
| | | | D2: MSBI1 761-5-1 (Ab 13) | DSM ACC3330 |

The localization of the antibody epitopes within MSBI1.176 Rep is shown in Fig. 8.

Anti-Rep antibodies of group A have an epitope within the amino acid sequence depicted in SEQ ID NO:3 (aa 198-217 of SEQ ID NO:1) and are capable of detecting MSBI1 Rep and Rep proteins comprising this conserved epitope of the BMMF1 group (e.g. MSBI2, CMI1, CMI2, CMI3, CMI4, HCBI4, HCBI6, C1MI.3M.1, C1MI.9M.1). In immunofluorescence assays such anti-Rep antibodies detect a specific Rep localization pattern, wherein the main localization is homogeneously distributed over the cytoplasm and nuclear membrane; and additional weak and homogeneously distributed localization is seen in the nucleus. An example of such a group A antibody is antibody AB01 523-1-1 (also called antibody 1-5; DSM ACC3327) which was employed in the examples as group A antibody.

Anti-Rep antibodies of group B have an epitope within the amino acid sequence depicted in SEQ ID NO:2 (aa 33-50 of SEQ ID NO:1) and are capable of detecting MSBI1 Rep and Rep proteins comprising this conserved epitope of the BMMF1 group (e.g. MSBI2, CMI1, CMI2, CMI3, CMI4, HCBI4, HCBI6, C1MI.3M.1, C1MI.9M.1). In immunofluorescence assays such anti-Rep antibodies detect specifically speckles (cytoplasmatic aggregations) of the Rep protein (often in the periphery of the nuclear membrane). An example of such a group B antibody is the antibody designated as AB02 304-4-1 (also called antibody 5-2; DSM ACC3328) which was employed in the examples as group B antibody.

Anti-Rep antibodies of group C detect specifically a structural epitope of MSBI1 (SEQ ID NO:1). In immunofluorescence assays such anti-Rep antibodies detect a specific Rep localization pattern, wherein the main localization is homogeneously distributed over the cytoplasm and nuclear membrane; and additional weak and homogeneously distributed localization is seen in the nucleus. An example of such a group C antibody is antibody MSBI1 381-6-2 (also called antibody 3-6 or antibody 3; DSM ACC3329) which was employed in the Example as group C antibody with an epitope in the sequence of aa 230-324. Another example of an antibody of a group C antibody is antibody MBSI1 572-13-19 (also called antibody 10-3 or antibody 10) detecting an epitope in the C-terminal domain of MSBI 1 Rep (aa 230-324). Another example of an antibody of a group C antibody is antibody MBSI1 617-1-3 (also called antibody 11-5) detecting an epitope in the N-terminal domain of MSBI 1 Rep (aa 1-136).

Anti-Rep antibodies of group D detect specifically a structural epitope of MSBI1 (SEQ ID NO:1), where antibody MSBI1 961-2-2 designated as "D1" (also called antibody 9-2; DSMACC3331) detects an epitope depicted in SEQ ID NO:9 (aa 281-287) in the C-terminal domain of MSBI1. Antibody MSBI1 761-5-1 (also called antibody 13; DSM ACC3328) designated as "D2" detects a 3D structural epitope of MSBI1 which is exclusively accessible under in vivo conditions and is not accessible in Western Blots. In immunofluorescence assays such anti-Rep antibodies detect specifically speckles (cytoplasmatic aggregations) of the Rep protein (often in the periphery of the nuclear membrane.

The availability of BMMF-specific Rep monoclonal antibodies and the demonstration of the DNA of these infectious agents in peritumour tissues, prompted the inventors to investigate patient tissue for the presence of specific structural components by immunogold electron microscopy using primary Rep monoclonal antibodies as tool. The identification of such gold-labelled structures in peritumour tissues of a pancreatic cancer patient is described in detail in the following examples.

The present ultrastructural analyses by immuno-electron microscopy (IEM) demonstrates BMMF Rep expression in macrophages in peritumour pancreatic cancer tissues. The gold decorated targets show a pleomorphic appearance, differing in size and shape and separate from the surrounding area. In the peritumour of pancreatic cancer, pleomorphic structures with diameters approximately between 50-200 nm were found. Cryo IEM as well as conventional resin TEM, which both allow for more delicate structural preservation compared to pre-embedding IEM of FFPE (formalin-fixed paraffin-embedded tissue) samples, show MSBI1.176 Rep overexpression-dependent segregation bodies in the cytoplasm of HEK293TT cells with potential to associate with membranes. In all modes of sample preparation, the detailed view into focal accumulations reveal ultrafine structures like voids and filaments indicative for higher order organization.

Semi-automated quantification of Rep, CD68, and CD163 immunofluorescence microscopic staining of individual FFPE tissues was used to compare the level Rep+, Rep+CD68+, Rep+CD68+CD163+ and CD68+ cells in the tissues of pancreatic cancer tumour and pathologist-validated tumour-adjacent tissues regions as well as in tissues from age-matched chronic pancreatitis patients and from age-matched non-tumour individuals. A significantly increased number of Rep+CD68+ cells (mean around 7.4% of all cells) was observed in the tumour of pancreatic cancer patients when compared to non-cancer healthy controls (mean <1%) and when compared to tumour-adjacent tissue (mean <1%). When specifying Rep-positive cells to the fraction of M2-like, CD163-positive macrophages within the population of all macrophages, an increased number of Rep+CD68+CD163+ cells (mean around 19.5% within all macrophages) was observed in the tumour of pancreatic cancer patients when compared to healthy controls (mean 5.5% of all macrophages), indicating that a large number of Rep+ Mfs is represented by M2-like macrophages stained triple-positive with Rep, CD68 and CD163 antibodies (Fig. 9). Cell-based quantification of the same parameters for chronic pancreatitis patients indicated that the amount of Rep+CD68+ cells (mean of around 4.0%) and of Rep+CD68+CD163+ cells within all macrophages (mean of around 9.5%) localized in between the values detected for pancreatic cancer tumour tissues (with highest values, 7.4% and 19.5%, respectively) and healthy controls (showing lowest values, <1% and 5.5%, respectively). Quantification of Rep+CD68+ cells and Rep+CD68+CD163+ cells within all macrophages in tumour-adjacent tissues (mean <1% and around 5.5%) was comparable to cell counts in healthy individuals. The numbers of Rep+, CD68+ and Rep+CD68+ cells were significantly increased in the peritumour when compared to respective quantifications of stained cells in the paired tumor (medians of 6.3% vs. 0.5% Rep+, 11.0% vs. 2.5% CD68+, 2.2% vs. <0.1% Rep+CD68+ cells, respectively) (Fig. 10).

Comparison Rep-stained area in Langerhans islets and staining intensity based on semi-automated quantification for tissues of healthy donors, PDAC patients and chronic pancreatitis patients (representative DAB IHC staining examples given in Figure 11) revealed significantly increased Rep-stained areas in Langerhans Islets of PDAC patients (approximately 38% mean) when compared to healthy controls (around 18% mean) (Fig. 12). Stained area in chronic pancreatitis (around 25% mean) was significantly smaller when compared to PDAC but still increased when compared to the group of healthy donors. The same trends were observed for Rep staining intensity, but on a generally higher level. Rep staining intensity was significantly increased in Langerhans Islets of PDAC patients (around 160 a.u., on average) when compared to healthy controls (around 135 a.u. on average). Staining intensity in chronic pancreatitis (around 140 a.u. on average) was significantly smaller when compared to PDAC, but still increased when compared to the healthy group.

The detection of Rep positivity in triple positive macrophages (Rep+CD68+CD163) was used to characterize the differentiation of Rep-positive macrophages and discriminate between a generally more M1-like (CD68+CD163-) or M2-like (CD68+CD163+) macrophage polarization. Quantification of triple positive macrophages might sharpen future diagnostic differentiation and help to identify (pathogenic) involvement of BMMF-positive macrophage populations to cancer. M1-like macrophages are expected to act pro-inflammatory (recruiting e.g. tumor-cell reactive lymphocytes) and act anti-tumorigenic while M2-like macrophages are considered pro-tumorigenic releasing cytokines and chemokines involved in recruitment of pro-tumorigenic regulatory T-cells.

The above-mentioned analyses may be seen as proof that the Rep Protein is a structural protein that can be targeted by antibodies, possibly both for preventive or therapeutic purposes.

In addition, the inventors have found a link between BMMF infection and type 2 diabetes since it has been shown by the inventors that BMMF infection is prevalent in insulin-producing pancreatic β-cells in the endocrine Langerhans Islets. It results in large aggregates of BMMF Rep protein that potentially interfere with insulin production. In later stages β-cell depletion occurs leading to the dependence on insulin injections. The link between diabetes type 2 and pancreatic cancer has been exemplified in Fig. 7.

The invention is further illustrated by, but not limited to, the following examples:

### EXAMPLES

### Example 1: Detection of BMMF protein targets in peritumor pancreatic cancer tissue

### Tissue staining

Peritumour pancreatic cancer tissues were obtained as FFPE section by the EPZ-Pancobank/BioMaterial Bank Heidelberg including patient consent and in accordance with the regulations of the tissue bank as approved by the Ethics Committee of Heidelberg University (Ethic votes S-708/2019 and S-083/2021).

**Table 2**

| **Antibody** | **Source** | **Host** | **Dilution** | **Final concentration in µg/ml** | **Incubation time** |
|---|---|---|---|---|---|
| **Primary** | | | | | |
| **Rep mAb #3-6** | **T. Bund, DKFZ** | **mouse** | **1:500** | **3.9** | **60 min at room temperature** |
| **Rep mAb #10-3** | **T. Bund, DKFZ** | **mouse** | **1:500** | **3.9** | |
| **CD68** | **Cell signaling #76437** | **Rabbit** | **1:1000** | **0.1** | |
| **insulin** | **Santa-Cruz, sc-8033** | **mouse** | **1:250** | **4** | **120 min** |

| For EM: | | | | | |
|---|---|---|---|---|---|
| **anti-mouse** | **DAKO, Z0259** | **rabbit** | **1:150** | | **60 min at room temperature** |

FFPE IHC DAB staining was performed based on the Zytomed ChemPlus (HRP) staining kit (Zytomed Systems) including epitope retrieval (30 min, 95 °C) with EDTA (pH 8.5, Sigma) as described previously (910 et al., 2021). Rep staining with AB3 (1 h, RT) was followed by colorization with the DAB high contrast kit (Zytomed Systems) and hematoxylin counterstain. Slides were scanned using a Hamamatsu Nanozoomer slide scanner (Hamamatsu) and analyzed with NDP.view2 Plus software (Hamamatsu).

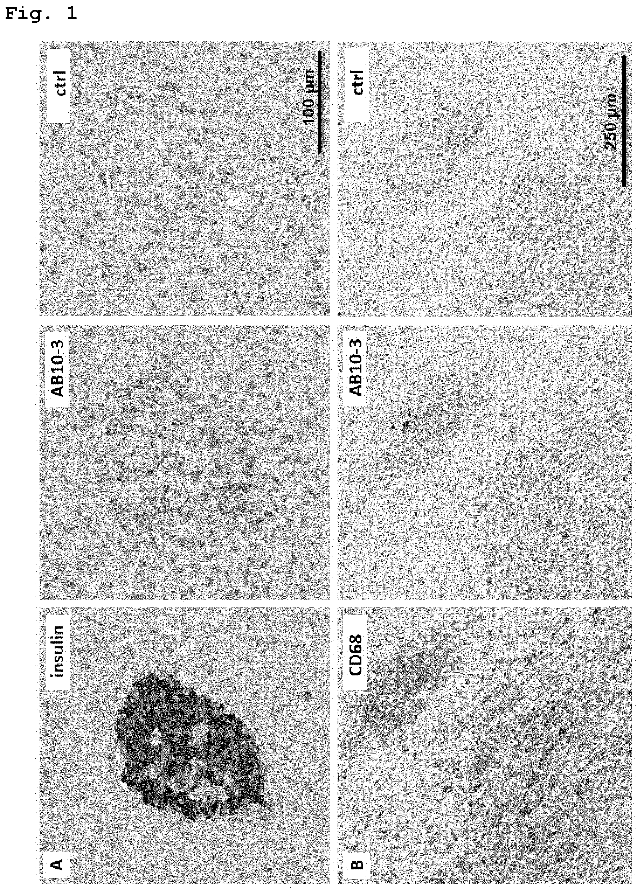

Staining of tissue PDAC tissue samples with anti-Rep antibodies (e.g. mAb 10; Table 1) shows detection of protein targets in peritumor tissue regions within pancreatic cancer patient samples. In general, the anti-Rep detection resulted in intense staining of smaller sized aggregated structures mainly within the cytoplasmic regions of insulin-positive beta-cells of the endocrine Langerhans Islands (Fig. 1, A). Additionally, a second staining pattern was observed in exocrine tissue regions. Here, a co-localization of the anti-Repstained signals with CD68-positive macrophages, which are distributed throughout the exocrine tissue regions and in the interstitium, was observed (Fig. 1, B). The regions with highest Rep-specific antibody detection correlate with regions with highest detection levels for CD68 positive cells pointing towards a localization of the Rep-specific antigens in inflammatory tissue areas, i.e. regions with especially high levels of inflammatory monocytes, circulating macrophages, or resident tissue macrophages. No signal detection was observed in control staining with an antibody isotype control.

### Example 2: Pre-embedding Immuno-Electron microscopy (IEM)

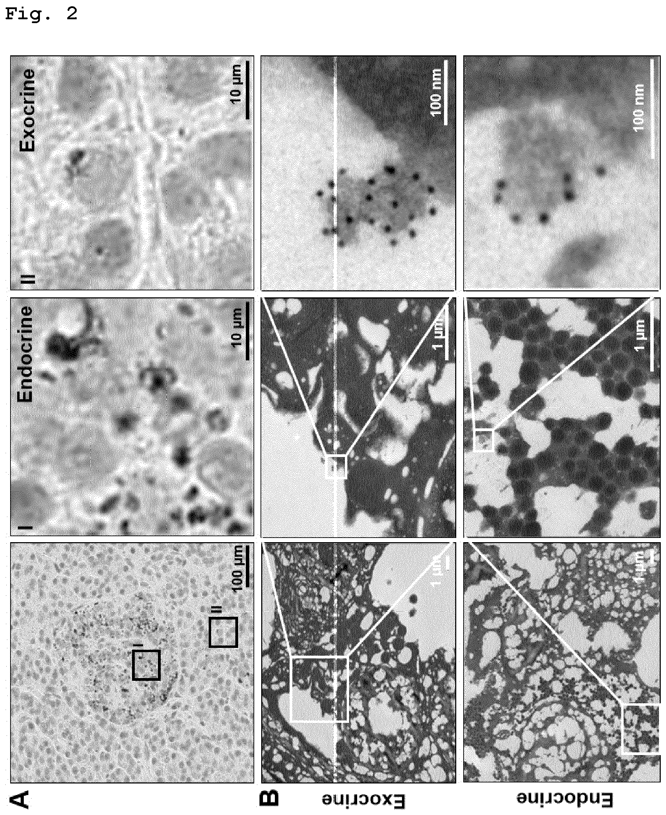

Tissue from a pancreatic cancer patient which stained positive for BMMF Rep in IHC was used to resolve the localization and physical expression of Rep targets at ultrastructural resolution. The inventors initially performed IHC DAB staining using monoclonal anti-BMMF1 Rep AB10 on one of the consecutive FFPE cuts to identify positive tissue regions (Fig. 2A). Based on Example 1, consecutive sections containing both exocrine and endocrine peritumour tissue regions were subsequently gold-labelled by pre-embedding IEM (pre-IEM) (Fig. 2B).

Therefore, standard procedures for immunolabelling of FFPE material followed by resin-embedding for subsequent ultrathin sectioning were as follows: FFPE-sections (3 µm thickness) mounted on glass slides were de-waxed with xylene and rehydrated in a graded series of ethanol. The immunoreaction was performed by incubation in 40 mM glycin (aldehyde block), 1% BSA (protein block), primary antibodies AB3 and polyclonal rabbit-anti-mouse (1:150, Z0259, Dako) as linker for the reporter Protein A-gold (CMC, nominal gold grain size 5 nm or 10 nm). Runs without primary antibody were included as reporter-only control. Sections on the glass slides were post-fixed in 2.5% glutaraldehyde, followed by 1% OsO4, dehydration through graded steps of ethanol and flat-embedding in epoxide (Serva). Ultrathin sections taken at nominal thickness 60 nm, were contrasted with uranyl and lead for direct visualization by electron microscopy.

Pre-embedding IEM staining revealed exclusive gold label at high density on distinct compact particulate structures, pleomorphic in size ranging between approximately 50-200 nm both in exocrine and endocrine tissue regions. The identified pleomorphic structures vary in size and shape and appear to contain filamentous substructures which are highlighted with arrowheads (Fig. 3). Apart from these structures, gold grains did not accumulate in other regions of the tissues. The cellular context of localization was not identified due to compromised structural preservation inherent to the FFPE protocol.

### Example 3: Cryo IEM (Tokuyasu method) after Overexpression of MSBI1.176 Rep in HEK293TT for validation of pre-IEM

### Cell culture and transfection

HEK293TT cells were cultured as described previously (Buck et al. 2005, de Villiers et al. 2011) and transfected with polyethylenimine (PEI, Sigma) at 70-80% confluency in 6-well plates with 1 µg of plasmid DNA per well for overexpression of either untagged H1MSB.1 Rep or 3xFlag-H1MSB.1 Rep protein (control: mock transfection with pcDNA(-)). Overexpression plasmids were designed based on PCR-cloning of the H1MSB.1 Rep gene (cloning via BamHI/KpnI, PCR primers: pcDNA3.1(-) H1MSB.1 Rep fwd: GC GGA TCC GCC ATG AGC GAC CTG ATC GTG AAA G (SEQ ID NO:21), rev: GC GGT ACC TCA AAA CAC GAC TCC AAA CTC TTC C (SEQ ID NO:22); pcDNA3.1(-) 3xFlag H1 MSB.1-Rep fwd: GT GGA TCC GCC ATG GAC TAC AAA GAC CAT GAC GGT GAT TAT AAA GAT CAT GAC ATC GAT TAC AAG GAT GAC GAT GAC AAG GGA GCAAGC GAC CTG ATC GTG AAA GAC AAT GC (SEQ ID NO:23), Rep rev: GA GGT ACC TCA AAA CAC GAC TCA AAC TCT TCC AGT TTA G (SEQ ID NO:24). Cells were harvested 48 hours after transfection. One part was analysed by SDS-PAGE and immunoblotting as described before (Bund et al. 2021) and the other part was fixed for cryo IEM (see below).

### Western blot analysis

Analysis by western blot was performed as described before (Bund et al. 2021), except that the cells were harvested after 48 h, followed by lysis in Lämmli buffer. Lysates were separated by SDS-PAGE on precast gels (Sigma True-PAGE 4-20%) prior to electro-transfer to nitrocellulose. Immunodetection by western blot was performed using two primary mouse monoclonal antibodies against Rep AB3 and AB7 (DKFZ Heidelberg (Bund et al. 2021)), respectively, or anti-Flag M2 (F3165, Sigma Aldrich) and by HRP-coupled goat anti-mouse IgG secondary antibody (115-035-205, Dianova).

Rep protein expression was verified by WB analysis using anti-Flag, as well as anti-Rep AB3 or AB7 antibodies. Full-length Rep protein bands of a molecular weight 40 kDa and 37 kDa for 3xFlag Rep and untagged Rep, respectively, were visible. In addition, smaller molecular weight bands, which had been described in a previous study (Bund et al. 2021), stained positive (Fig. 4)

### Cryo IEM (Tokuyasu method)

We followed the protocol of Slot & Geuze (Slot and Geuze 2007). HEK293TT cells transfected with the respective plasmids were fixed in buffered aldehyde (2% formaldehyde, 0.2% glutaraldehyde, 100 mM phosphate buffer, pH 7.2) and embedded in 10% gelatine. Vibratome-sections (200 µm thickness) of patient biopsies or blocks of gelatine-embedded cells were perfused with 2.3 M sucrose in 50 mM phosphate buffer and frozen in liquid nitrogen. Cryo sections with nominal thickness of 60 nm were cut at 160 K with an UCT7/FC7 cryo-ultramicrotome (Leica). Immunoreactions were performed on ice after buffer wash and incubation in 40 mM glycine and 1% BSA. To enable binding of protein A-gold reporter (1:50, CMC, nominal size of gold grains: 5 nm or 10 nm), a polyclonal rabbit anti-mouse antibody (1:150, Z0259, DAKO) was used to bridge the primary antibodies of murine origin. Control samples included a reporter-only (without primary antibody), or staining either with primary antibody isotype IgG1 (MG1-45, Biolegend) or anti-Flag.

Overexpression of both constructs led to the formation of large segregation complexes in the cytoplasm of transfected cells (Fig. 5). These complexes stained positive (with anti-Rep AB3, AB7 and anti-Flag, respectively), whereas the residual cytoplasm remained gold label-free. Untagged H1MSB.1 Rep segregated in addition in a nucleoplasmic location (Fig. 6A) and showed an association with membranes (Fig. 6B). An isotype antibody did not react with the target structures (Fig. 5). Transfection with a pcDNA3.1 (-) vector (no Rep insert) did not result in segregation structures.

### Resin TEM (without immunolabelling)

Transfected HEK293TT cells expressing H1MSB.1 Rep and cultivated on punched Aklar-Fluoropolymer films (EMS) were resin-embedded for ultrathin sectioning according to standard procedures. This included primary fixation in buffered aldehyde (4% formaldehyde, 2% glutaraldehyde, 1 mM CaCl2, 1 mM MgCl2 in 100 mM Nacacodylate, pH 7.2), post-fixation in buffered 1% osmium tetroxide and en-block staining in 1% uranylacetate, dehydration in graded steps of ethanol and embedding in epoxide (glycidether, NMA, DDSA; Serva). Ultrathin sections, with nominal thickness 60 nm and contrast-stained with lead-citrate and uranylacetate, were analysed with EM 910 at 80 kV (Carl Zeiss) and micrographs taken using a slow scan CCD camera (TRS).

Overexpression of non-tagged MSBI1.176 Rep led to the formation of large segregation complexes in the cytoplasm of transfected cells not observed after mock transfection with pcDNA3.1 (-). The segregation structures showed an association with membranes (see asterisks, Fig. 6C).

### Example 4: Cell-based quantification of Rep-, CD68- and CD163-positive cells in tumour and peritumour tissues of pancreatic ductal adenocarcinoma patients, chronic pancreatitis patients and non-cancer controls by immunofluorescence microscopy

### Tissue Staining and Tissue Analysis

Tissues from pancreatic cancer patients, patients with chronic pancreatitis and tissues from healthy transplant donors were obtained as FFPE sections the PancoBank of European Pancreas Center (EPZ) Heidelberg, Germany in accordance with the regulations of the Tissue Bank which include patient consent and approval by the Ethics committee of the Heidelberg University (ethics vote S-708/2019) and in full concordance to the standards set by the Declaration of Helsinki. FFPE sections were stained with primary anti-BMMF1 Rep (monoclonal AB3 and AB10, DKFZ Heidelberg, 1:1 mix), anti-CD68 (Cell signaling # 76437) and anti-CD163 (Novusbio, NB110-40686) antibodies overnight at room temperature in a dilution of 1:250 (Rep), 1:500 (CD68) and 1:200 (CD163) as described in Bund et al., 2021. Secondary goat anti-mouse Alexa Fluor 594 (Invitrogen #A 11032), goat anti-rabbit Alexa Fluor 488 (Invitrogen, #A11034) and donkey anti-mouse Alexa Fluor 647 (Invitrogen #A31571) were incubated for 60 min at room temperature. For negative control staining, PBT diluent containing PBS, BSA, Tween20 and sodiumazide was used. Slides were scanned with a Hamamatsu Nanozoomer slide scanner (Hamamatsu) and analyzed with NDP.view2 Plus software (Hamamatsu).

Semi-automated quantification of Rep, CD68, CD163 staining of individual FFPE tissues was performed with an in-house developed script for ImageJ/Fiji (v1.52) based on signal intensity of DAPI nuclear and antibody staining of cells in digitalized scans after Rep/CD68/CD163 co-immunodetection. Cell density was measured in 8-12 randomly selected microscopic areas (around 15,000 cells per sample, in total). The data was represented as % average number (mean) of Rep, CD68, CD163 positive cells and/or its combinations normalized either to total number of nuclei (or total number of CD68+ macrophages) as described in Bund et al., 2021 and Nikitina et al, 2023 for similar quantification in colorectal cancer context.

The results are shown in Fig. 9 and 10.

**Table 3**

| **Antibody** | **Source** | **Host** | **Dilution** | **Final concentration in µg/ml** | **Incubation time** |
|---|---|---|---|---|---|
| | | | | | |
| **Rep mAb #3-6** | **T. Bund, DKFZ** | **mouse** | **1:1000** | **2** | **60 min at room temperature** |
| **Rep mAb #10-3** | **T. Bund, DKFZ** | **mouse** | **1:1000** | **2** | |
| **CD68** | **Cell signaling #76437** | **Rabbit** | **1:1000** | **0.1** | |
| **CD163** | **Novusbio, Centennial, CO, USA, NB110-40686** | **Mouse** | **1:200** | **5** | |
| **Insulin** | **Santa Cruz Biotechnolo gy, Dallas, Texas, sc-8033** | **Mouse** | **1:200** | **1** | **180 min at room temperature** |

### Example 5: Semi-automated quantification of Rep+Insulin+ cells in tumour and peritumour tissues of pancreatic ductal adenocarcinoma patients, chronic pancreatitis patients and non-cancer controls by immunofluorescence microscopy

### Tissue Staining and Tissue Analysis

Tissues (same as described in Example 4) were stained with primary anti-BMMF1 Rep (monoclonal AB10, DKFZ Heidelberg anti-BMMF1 Rep (monoclonal AB10, DKFZ Heidelberg, 1:500 dilution, 30 min incubation at room temperature) and anti-Insulin antibody (in consecutive tissue section to allocate β-cell island, Santa Cruz Biotechnology, Dallas, Texas, sc-8033, mouse, 1:200, 180 min at RT). Secondary rabbit anti-mouse (Abcam, Cambridge, UK, #125904) was incubated for 20 min at room temperature. Detection was performed by using Bond Polymer Refine Detection Kit (Leica Biosystems, #DS9800) including DAB chromogen and hematoxylin counterstain. Both DAB brightfield and immunofluorescent slides were scanned with a Hamamatsu Nanozoomer slide scanner (Hamamatsu, Shizuoka, Japan) and analyzed with ndp.view2 plus software (Hamamatsu).

For the analyses of Rep antibody-stained positive area and staining intensity of pancreatic islets, semi-automated quantification Rep staining was performed with an in-house developed script for ImageJ/Fiji (v1.52) based on DAB signal area and mean intensity in each selected islets of tissue. 20-40 islets per tissue (maximum number available) were included into analyses. The data was represented as % Rep-stained area in Langerhans islets (mean) and Rep staining intensity in Langerhans islets as described in Nikitina et al., 2022.

The results are shown in Fig. 11 (representative DAB IHC tissue staining) and Fig. 12.

### SEQUENCE SUMMARY

| SEQ ID NO | SEQUENCE |
|---|---|
| | Amino acid sequence of Rep protein encoded by MSBI1.176 |
| 1 | MSDLIVKDNALMNASYNLALVEQRLILLAIIEARETGKGINANDPLTVHASSYINQFN VERHTAYQALKDACKDLFARQFSYQEKRERGRINITSRWVSQIGYMDDTATVEIIFAP AVVPLITRLEEQFTQYDIEQISGLSSAYAVRMYELLICWRSTGKTPIIELDEFRKRIG VLDTEYTRTDNLKMRVIELALKQINEHTDITASYEQHKKGRVITGFSFKFKHKKQNSD KTPKNSDSSPRIVKHSQIPTNIVKQPENAKMSDLEHRASRVTGEIMRNRLSDRFKQGD ESAIDMMKRIQSEIITDAIADQWESKLEEFGVVF |
| 2 | Amino acid sequence of Rep peptide fragment |
| | EARETGKGINANDPLTVH |
| 3 | Amino acid sequence of Rep peptide fragment |
| | KQINEHTDITASYEQHKKGRT |
| 4 | His-Tag (with two neutral stuffer amino acids) |
| | GAHHHHHH |
| 5 | T7-Tag |
| | MASMTGGQQMG |
| 6 | FLAG-Tag |
| | DYKDDDDK |
| 7 | Strep-II-Tag |
| | WSHPQFEK |
| 8 | Amino acid sequence of Rep protein encoded by MSBI2.176 |
| | MSKLVVKDNALMNASYNLDLVEQRLILLAIIEARESGKGINANDPLTVHA ESYINQFGVHRVTAYQALKDACDNLFARQFSYQSKSEKGNIQNHRSRWVS EIIYIDTEATVKIIFAPAIVPLITRLEEQFTKYDIEQISDLSSAYAIRLY ELLICWRSTGKTPIIGLGEFRNRVGVLDSEYHRIAHLKERVIEHSIKQIN EHTDITATYEQHKKGRTITGFSFKFKQKKPKQAEIATETPKTATNDPDTT KPLTEPQIAKYSMILCKLGSISDLSNFPDYPAFANWIGNILRNPEKADEQ IAKRIFTALKTETDYSKKN |
| 9 | MSBI.1 specific epitope |
| | NRLSDRF |
| 10 | Amino acid sequence of Rep protein encoded by CMI1.252 |
| | MSDLIVKDNALMNASYNLALVEQRLILLAILEARETGKGINANDPLTVHASSYINQFN VERHTAYQALKDACKDLFARQFSYQEKRERGRINITSRWVSQIGYMDDTATVEIIFAP |
| | AVVPLITRLEEQFTQYDIEQISELSSAYAVRLYELLICWRSTGKTPIIDLTEFRKRLG VLDTEYTRTDNLKMRVIELGLKQINEHTDITASYEQHKKGRTITGFSFKFKQKKKTGA EMPKNSDSSPHIEKPSQIPANIAKQPENAKKDDLGHRASKITGLIMSNGLADRFKRGD ESVIDMMKRIKEEITTDTTADQWENKLEEFGVIFQS |
| 11 | Amino acid sequence of Rep protein encoded by CMI2.214 |
| | MSDLIVKDNALMNASYNLDLVEQRLILLAILEARETGKGINANDPLTVHAESYINQFG VARQTAYQALKDACKDLFARQFSYQEKRERGRANITSRWVSQIAYIDETATVEVIFAP AVVPLITRLEEQFTQYDIEQISGLSSAYAVRLYELLICWRSTGKTPVIELAEFRKRLG VLNDEYTRSDNFKKWIIENPIKQINEHTDITASYEQHKKGRTITGFSFKFKQKKKTEP ETPKNSDSSQRIEKPSQIPANIVKQPENANLSDLQHRASKITGLIMSNRLSDRFKQGD ESIMQMMARIQSEITTDSIADQWQSKLEEFGVVF |
| 12 | Amino acid sequence of Rep protein encoded by CMI3.168 |
| | MSDLIVKDNALMNASYNLALVEQRLILLAILEARETGKGINANDPLTVHASSYINQFN VERHTAYQALKDACKDLFARQFSYQEKRERGRANITSRWVSQIAYIDETATVEVIFAP AVVPLITRLEEQFTQYDIEQISGLSSAYAVRLYELLICWRTTGKTPVLDLTEFRKRLG VLDTEYTRTDNLKMRVIEQSLKQINKHTDITASYEQHKKGRTITGFSFKFKQKKKTEP ETPKNNDSGVSKPKTVEIPAEVVKQPKNTNLSDLEKRVRMITGAIAKNNLASRFQHGN ESPLDMMKRIQSEITSDETADLWQNKLESMGVVF |
| 13 | DNA sequence MSBI1 Rep codon-optimized |
| | ATGAGCGACCTGATCGTGAAAGACAATGCCCTGATGAACGCCTCCTACAACCTGGCAC TGGTCGAACAGAGACTGATTCTGCTGGCTATCATCGAGGCAAGGGAGACCGGCAAGGG CATCAACGCCAATGACCCCCTGACAGTGCACGCCAGCTCCTACATCAACCAGTTTAAT GTGGAGCGCCACACCGCCTATCAGGCCCTGAAGGACGCCTGCAAGGATCTGTTTGCCC GGCAGTTCAGCTACCAGGAGAAGCGGGAGAGAGGCAGGATCAACATCACAAGCAGATG GGTGTCCCAGATCGGCTATATGGACGATACCGCCACAGTGGAGATCATCTTTGCACCA GCAGTGGTGCCTCTGATCACCAGGCTGGAGGAGCAGTTCACACAGTACGACATCGAGC AGATCTCCGGACTGTCTAGCGCCTACGCCGTGCGCATGTATGAGCTGCTGATCTGTTG GCGGTCTACCGGCAAGACACCTATCATCGAGCTGGATGAGTTCCGCAAGCGGATCGGC GTGCTGGACACCGAGTACACCAGAACAGATAACCTGAAGATGAGAGTGATCGAGCTGG CCCTGAAGCAGATCAATGAGCACACCGATATCACAGCCTCTTATGAGCAGCACAAGAA GGGCCGCGTGATCACCGGCTTCAGCTTTAAGTTCAAGCACAAGAAGCAGAACTCTGAC AAGACACCAAAGAATAGCGATTCCTCTCCCCGGATCGTGAAGCACAGCCAGATCCCTA CCAACATCGTGAAGCAGCCAGAGAATGCCAAGATGTCCGACCTGGAGCACAGGGCATC TAGGGTGACAGGCGAGATCATGAGAAATAGGCTGAGCGATCGGTTCAAGCAGGGCGAC GAGTCCGCCATCGATATGATGAAGAGAATCCAGTCCGAGATCATCACCGACGCCATCG CCGATCAGTGGGAATCTAAACTGGAAGAGTTTGGAGTCGTGTTTGGAGCACATCACCA TCATCATCACTGA |
| 14 | Protein sequence MSBI1 Rep codon-optimized |
| | MSDLIVKDNALMNASYNLALVEQRLILLAIIEARETGKGINANDPLTVHASSYINQFN VERHTAYQALKDACKDLFARQFSYQEKRERGRINITSRWVSQIGYMDDTATVEIIFAP |
| | AVVPLITRLEEQFTQYDIEQISGLSSAYAVRMYELLICWRSTGKTPIIELDEFRKRIG VLDTEYTRTDNLKMRVIELALKQINEHTDITASYEQHKKGRVITGFSFKFKHKKQNSD KTPKNSDSSPRIVKHSQIPTNIVKQPENAKMSDLEHRASRVTGEIMRNRLSDRFKQGD ESAIDMMKRIQSEIITDAIADQWESKLEEFGVVFGA |
| 15 | DNA sequence MSBI1 Rep wild-type |
| | ATGAGCGATTTAATAGTAAAAGATAACGCCCTAATGAATGCTAGTTATAACTTAGCTT TGGTTGAACAGAGGTTAATTCTATTAGCAATCATAGAAGCGAGAGAAACAGGCAAAGG GATTAATGCCAATGATCCTTTAACAGTTCATGCAAGTAGCTATATCAATCAATTTAAC GTAGAAAGGCATACGGCATATCAAGCCCTCAAAGATGCTTGTAAAGACTTGTTTGCCC GTCAATTCAGTTACCAAGAAAAGCGAGAACGAGGACGAATTAATATTACAAGTCGATG GGTTTCGCAAATTGGCTATATGGACGATACAGCAACCGTTGAGATTATTTTTGCCCCT GCGGTTGTTCCTCTGATTACACGGCTAGAGGAACAGTTCACCCAGTACGATATTGAGC AAATTAGCGGTTTATCGAGTGCATATGCTGTTCGTATGTACGAACTGCTGATTTGTTG GCGTAGCACAGGCAAAACACCAATTATTGAGCTAGACGAGTTTAGAAAGCGAATAGGT GTTTTAGATACTGAATACACTAGAACAGATAATTTAAAGATGCGAGTTATTGAATTAG CCCTAAAACAAATCAACGAACATACAGACATCACAGCAAGCTATGAACAACACAAAAA AGGGCGAGTGATTACAGGATTCTCATTCAAGTTTAAGCACAAGAAACAAAACAGCGAT AAAACGCCAAAAAATAGCGATTCTAGCCCACGTATCGTAAAACATAGTCAAATCCCTC CAACATTGTAAAACAGCCTGAAAACGCCAAAATGAGCGATTTAGAACATAGAGCGAGC CGTGTTACAGGGGAAATAATGCGAAATCGTCTGTCAGATCGGTTTAAACAAGGCGATG AATCAGCAATCGACATGATGAAACGTATTCAAAGTGAAATAATAACCGATGCAATAGC AGACCAGTGGGAAAGCAAACTGGAGGAGTTTGGCGTGGTTTTTTAG |
| 16 | Amino acid sequence of Rep protein encoded by CMI4.158 |
| | MSDLIVKDNALMNASYNLALVEQRLILLAILEARETGKGINAND PLTVHANSYINQFNVERHTAYQALKDACKDLFARQFSYQEKRERGRINITSRWVSQIG YMDDTATVEVIFAPAVVPLITRLEEQFTQYDIEQISGLSSAYAVRLYELLICWRSTGK TPVIELAEFRKRIGVLDTEYTRTDNLKMRVIELALKQINEHTDITASYEQHKKGRTIT GFSFKFKQKKKTELETPKNSDSSPHIEKPSQIPTNIVKQPENAKKDDLGHRASKITGL IMSNGLADRFKRGDESVMNMMKRIKEEITTDATADLWQNKLESMVWFFSHD |
| 17 | Amino acid sequence of Rep protein encoded by HCBI3 |
| | MYRWFMSDLIVKDNALMNASYNLDLVEQRLVLLAILEARESGKGINANDP LTVHAESYINQFGVARQTAYQALKGACKDLFARQFSYQEKRERGRANITS RWVSQIAYIDETATVEVIFAPAVVPLITRLEEQFSKYDIEQISSLSSAYA VRLYELLICWRTTGKTPVIDLTEFRKRIGVLDTEYQRMDVFKRGVLELAL KQVNEHTDITATYEQHKKGRVITGFSFKFKQKKPKQAEIATETPKTATTD LATTKPLTEPQIAKYSMILCKLGSISDLGNFPDYPTFANWIANILRTPEK ADEQIAKRIFTALKTETDYSKKN |
| 18 | Amino acid sequence of Rep protein encoded by HCBI4 |
| | MKNGLVVKDNALMNASYNLEVTEQRLILLAIISARETGQGITSD SKLEIHASDYAIQFSVAKETAYEALKSAVNNLFERQFSFREETKKGTGIVRSRWVSRI KYIDDAAILEITFAPDVVPLITRLEEHFTSYQIKQIAQLTSKYAIRLYELLIAWRTTG KVPELELSEFRNRLGIASNEYTAMNNFKSRVLEPSIKQINEHTDITVTYEQHKKGRTI TGFSFRFKQKQQAKKIETNRDPNTPDFFIKMTDAQRHLFANKMSEMPEMGKYSQGTES YQQFAIRIADMLLQPEKFRELYPILEKAGFKE |
| 19 | Amino acid sequence of Rep protein encoded by C1MI.3M.1 |
| | MSELIVKDNALMNASYNLDLVEQRLILLAILEARESGKGINANDPLTVHA ESYINQFGVHRTTAYQALKDACKDLFARQFSYQEKRERGRANITSRWVSQ IAYIDETGTVEVIFAPAVVPLITRLEEQFTKYDIEQISGLSSAYAVRLYE LLICWRTTGKTPIIDLTEFRKRIGVLDTEYQRMDVFKRGVLELALKQVNE HTDITASYEQHKKGRVITGFSFKFKQKKPKQAEIATETPKTATNDPDTTK PLTEPQIAKYSMILCKLGSISDLSNFPDYPAFANWIGNILRNPEKADEQI AKRIFTALKTETDYSKKN |
| 20 | Amino acid sequence of Rep protein encoded by C1MI.9M.1: |
| | MRLMSELVVKDNALMNASYNLDLVEQRLILLAIVEARESGKGIN ANDPLTVHAESYINQFGVARQTAYQALKDACDDLFARQFSYQEINERGNTENVRSRWV SEVRYVDAEATVKLIFAPIVIPLITRLEERFTQYEMKQISELSTGYAIRLYELLICWR ATGKTPVIELADFRQRMGVLDSEYQRMYDLKKYVLEPSLKQINEHTDITATYEQHKKG RVITGFSFKFKQKKPKQAEIATETPKTATNDQNTIKPLTEPQIAKYSMILCKLGSISD LSNFPDYPSFANWIANILRTPEKADEQAAKRIFTALKTETDYSKKN |

### REFERENCES:

Eilebrecht, S., et al. (2018),"Expression and replication of virus-like DNA in human cells", Scientific Reports 8:2851
Funk, M., et al. (2014). "Isolation of protein-associated circular DNA from healthy cattle serum". Genome Announc 2(4)
Giraldo, R., et al. (2011). "RepA-WH1 prionoid: a synthetic amyloid proteinopathy in a minimalist host." Prion 5(2):60-64
Gunst, K., et al. (2014). "Isolation of bacterial plasmid-related replication-associated cirular DNA from a serum sample of a multiple sclerosis patient." Genome Announc 2(4).
Lamberto, I., et al. (2014). "Mycovirus-like DNA virus sequences from cattle serum and human brain and serum samples from multiple sclerosis patients." Genome Announc 2(4).
Manuelidis L., 2011. "Nuclease resistant circular DNAs co-purify with infectivity in scrapie and CJD". J. Neurovirol. 17:131-145.
Torreira, E., et al. (2015). "Amyloidogenesis of bacterial prionoid RepA-WH1 recaptiulates dimer to monomer transitions of RepA in DNA replication initiation." Structure 23(1):183-189
Whitley, C., et al. (2014). "Novel replication-competent cirulara DNA molecules from healthy cattle serum and milk and multiple sclerosis-affected human brain tissue." Genome Announc 2(4).
zur Hausen, H. and de Villiers, E.-M. (2015). "Dairy cattle serum and milk factors contributing to the risk of colon and breast cancers." Int. J. Cancer 137: 959-967
zur Hausen, H., Bund, T., de Villiers, E.-M. (2017). "Infectious agents in bovine red meat and milk and their potential role in cancer and other chronic diseases." Curr. Top.Microbiol. Immunol., Volume 407, 83-116.
Bund, T., E. Nikitina, D. Chakraborty, C. Ernst, K. Gunst, B. Boneva, C. Tessmer, N. Volk, A. Brobeil, A. Weber, M. Heikenwalder, H. zur Hausen and E. M. de Villiers (2021). Analysis of chronic inflammatory lesions of the colon for BMMF Rep antigen expression and CD68 macrophage interactions. Proc Natl Acad Sci U S A 118(12):e2025830118. doi: 10.1073/pnas.2025830118.
Slot, J. W. and H. J. Geuze (2007). Cryosectioning and immunolabeling. Nat Protoc 2(10): 2480-2491.
Falida, K., S. Eilebrecht, K. Gunst, H. zur Hausen and E. M. de Villiers (2017). Isolation of Two Virus-Like Circular DNAs from Commercially Available Milk Samples. Genome Announc 5(17):e00266-17. doi: 10.1128/genomeA.00266-17.
König, M. T., R. Fux, E. Link, G. Sutter, E. Märtlbauer and A. Didier (2021a). Circular Rep-Encoding Single-Stranded DNA Sequences in Milk from Water Buffaloes (Bubalus arnee f. bubalis). Viruses 13(6):1088. doi: 10.3390/v13061088.
König, M. T., R. Fux, E. Link, G. Sutter, E. Märtlbauer and A. Didier (2021b). Identification and Characterization of Circular Single-Stranded DNA Genomes in Sheep and Goat Milk. Viruses 13(11):2176. doi: 10.3390/v13112176.
De Villiers and zur Hausen (2021), Bovine Meat and Milk Factors (BMMFs): Their proposed role in common human cancers and type 2 diabetes mellitus, Cancers (Basel), 13(21): 5407
De Villiers, E.M., Gunst, K., Chakraborty D., Ernst, C., Bund, T., zur Hausen, H., (2019), A specific class of infectious agents isolated from bovine serum an diary products and peritumoral colon cancer tissue, Emerging Microbes and Infections 8(1), 1205-1218
Nikitina, E., Burk-Körner, A., Wiesenfarth, M., Alwers, E., Heide, D., Tessmer, C., Ernst, C., Krunic, D., Schrotz-King, P., Chang-Claude, J., von Winterfeld, M., Herpel, E., Brobeil, A., Brenner, H., Heikenwalder, M., Hoffmeister, M., Kopp-Schneider, A., Bund, T., Bovine meat and milk factor protein expression in tumor-free mucosa of colorectal cancer patients coincides with macrophages and might interfere with patient survival. Molecular Oncology, 2023. DOI: 10.1002/1878-0261.13390
Nikitina, E., Alikhanyan, K., Nessling, M., Richter, K., Kaden, S., Ernst, C., Seitz, S., Chuprikova, L., Haefele, L., Gunst, K., Rahbari, N., Birgin, E., Rasbach, E., Rahbari, M., Brobeil, A., Schenk, M., Büchler, M., de Villiers, E.-M., Bund, T.*, zur Hausen, H. Structural Expression of BMMF in tissues of colorectal, lung and pancreatic cancer patients. International Journal of Cancer, 2022. DOI: 10.1002/ijc.34374

## Claims

1. An in-vitro use of Bovine Meat and Milk Factor Group 1(BMMF1) Rep Protein as a diagnostic marker for pancreatic cancer.

2. The in-vitro use of claim 1, wherein the Rep protein is a MSBI1 genome-encoded Rep protein (MSBI1 Rep), a MSBI2 genome-encoded Rep protein (MSBI2 Rep), a CMI1 genome-encoded Rep protein (CMI1 Rep), a CMI2 genome-encoded Rep protein (CMI2 Rep), CMI3 genome-encoded Rep protein (CMI3 Rep) or CMI4 genome-encoded Rep protein (CMI4 Rep).

3. An in-vitro method for providing a diagnosis or determining a predisposition for pancreatic cancer in a subject, comprising the step of
detecting Rep protein in an isolated sample obtained from a subject by using anti-Rep antibodies that bind to an epitope comprised by SEQ ID NO:2 or SEQ ID NO:3.

4. The in-vitro method of claim 3, wherein the antibody specific for Rep protein binds to an epitope that is within an amino acid sequence selected from the group consisting of amino acids from 1 to 136, from 137 to 229 and from 230 to 324 of SEQ ID NO:1.

5. The in-vitro method of claim 3 or 4, wherein the sample from a subject is selected from the group consisting of a cancerous pancreatic tissue, peripheral tissue surrounding the cancerous tissue, (benign) cysts.

6. The in-vitro method of any of claims 3 to 5, wherein additionally CD68 positive cells are detected in the sample by an anti-CD68 antibody.

7. The in-vitro method of any of claims 3 to 6, wherein additionally CD163 positive cells are detected in the sample by an anti-CD163 antibody.

8. The in-vitro method of any of claims 3 to 7, wherein additionally insulin positive cells are detected in the sample by an anti-insulin antibody.

9. An in-vitro use of Bovine Meat and Milk Factor Group 1(BMMF1) Rep Protein as a diagnostic marker for diabetes.

## Patentansprüche

1. In-vitro Verwendung des Rep-Proteins der Rindfleisch- und Milchfaktorgruppe 1 (BMMF1) als ein diagnostischer Marker für Bauchspeicheldrüsenkrebs.

2. In-vitro Verwendung nach Anspruch 1, wobei das Rep-Protein ein MSBI1-genomkodiertes Rep-Protein (MSBI1 Rep), ein MSBI2-genomkodiertes Rep-Protein (MSBI2 Rep), ein CMI1-genomkodiertes Rep-Protein (CMI1 Rep), ein CMI2-genomkodiertes Rep-Protein (CMI2 Rep), ein CMI3-genomkodiertes Rep-Protein (CMI3 Rep) oder ein CMl4-genomkodiertes Rep-Protein (CMI4 Rep) ist.

3. In-vitro Verfahren zur Bereitstellung einer Diagnose oder Feststellung einer Veranlagung für Bauchspeicheldrüsenkrebs bei einem Patienten, umfassend den Schritt des
Nachweisens des Rep-Proteins in einer isolierten Probe, die mittels Anti-Rep-Antikörpern, die an ein Epitop binden, das von SEQ ID NO: 2 oder SEQ ID NO: 3 umfasst ist, von einem Patienten erhalten wird.

4. In-vitro Verfahren nach Anspruch 3, wobei der für das Rep-Protein spezifische Antikörper an ein Epitop bindet, das sich in einer Aminosäuresequenz befindet, die aus der Gruppe bestehend aus den Aminosäuren 1 bis 136, 137 bis 229 und 230 bis 324 der SEQ ID NO: 1 ausgewählt ist.

5. In-vitro Verfahren nach Anspruch 3 oder 4, wobei die Probe aus einem Patienten aus der Gruppe bestehend aus krebsartigem Bauchspeicheldrüsengewebe, peripherem Gewebe, das das krebsartige Gewebe umgibt, (gutartigen) Zysten ausgewählt ist.

6. In-vitro Verfahren nach einem der Ansprüche 3 bis 5, wobei in der Probe zusätzlich CD68-positive Zellen mittels eines Anti-CD68-Antikörpers nachgewiesen werden.

7. In-vitro Verfahren nach einem der Ansprüche 3 bis 6, wobei in der Probe zusätzlich CD163-positive Zellen mittels eines Anti-CD163-Antikörpers nachgewiesen werden.

8. In-vitro Verfahren nach einem der Ansprüche 3 bis 7, wobei in der Probe zusätzlich Insulin-positive Zellen mittels eines Anti-Insulin-Antikörpers nachgewiesen werden.

9. In-vitro Verwendung des Rep-Proteins der Rindfleisch- und Milchfaktorgruppe 1 (BMMF1) als ein diagnostischer Marker für Diabetes.

## Revendications

1. Utilisation in-vitro de protéine Rep du groupe 1 des facteurs de viande et de lait bovins (BMMF1) en tant que marqueur de diagnostic pour le cancer du pancréas.

2. Utilisation in-vitro selon la revendication 1, dans laquelle la protéine Rep est une protéine Rep codée par le génome MSBI1 (Rep-MSBI1), une protéine Rep codée par le génome MSBI2 (Rep-MSBI2), une protéine Rep codée par le génome CMI1 (Rep-CMI1), une protéine Rep codée par le génome CMI2 (Rep-CMI2), une protéine Rep codée par le génome CMI3 (Rep-CMI3) ou une protéine Rep codée par le génome CMI4 (Rep-CMI4).

3. Procédé in-vitro pour fournir un diagnostic du cancer du pancréas ou pour déterminer une prédisposition à celui-ci chez un sujet, comprenant l'étape suivante :
la détection d'une protéine Rep dans un échantillon isolé provenant d'un sujet au moyen d'anticorps anti-Rep qui se lient à un épitope compris dans le SEQ ID n°2 ou le SEQ ID n°3.

4. Procédé in-vitro selon la revendication 3, dans lequel l'anticorps spécifique à une protéine Rep se lie à un épitope qui se trouve à l'intérieur d'une séquence d'acides aminés choisie parmi le groupe comprenant des acides aminés de 1 à 136, de 137 à 229 et de 230 à 324 du SEQ ID n° 1.

5. Procédé in-vitro selon la revendication 3 ou 4, dans lequel l'échantillon provenant d'un sujet est sélectionné dans le groupe comprenant un tissu pancréatique cancéreux, un tissu périphérique entourant le tissu cancéreux et des kystes (bénins).

6. Procédé in-vitro selon l'une quelconque des revendications 3 à 5, dans lequel en outre des cellules positives au CD68 sont détectées dans l'échantillon par un anticorps anti-CD68.

7. Procédé in-vitro selon l'une quelconque des revendications 3 à 6, dans lequel en outre des cellules positives au CD163 sont détectées dans l'échantillon par un anticorps anti-CD163.

8. Procédé in-vitro selon l'une quelconque des revendications 3 à 7, dans lequel en outre des cellules positives à l'insuline sont détectées dans l'échantillon par un anticorps anti-insuline.

9. Utilisation in-vitro de protéine Rep du groupe 1 des facteurs de viande et de lait bovins (BMMF1)) en tant que marqueur de diagnostic pour le diabète.
